# EUROPEAN PATENT APPLICATION

(11) **EP 4 393 947 A1**
(43) Date of publication of application: **03.07.2024**
(21) Application number: 22861170.3
(22) Date of filing: 15.08.2022
(51) Int. Cl.: C07K 16/18, C07K 14/47

(54) **ANTI-PD-1 SIGNAL PEPTIDE ANTIBODY AND USE THEREOF**

(30) Priority: 25.08.2021 JP 2021136983
(71) Applicant: Toagosei Co., Ltd., Minato-ku Tokyo 105-8419 (JP)
(72) Inventor: BAILEYKOBAYASHI, Nahoko, Tsukuba-shi, Ibaraki 300-2611 (JP); YOSHIDA, Tetsuhiko, Tsukuba-shi, Ibaraki 300-2611 (JP)
(74) Representative: Bals & Vogel Patentanwälte PartGmbB
(86) International application number: PCT/JP2022/030842
(87) International publication number: WO 2023/026881

(57) **Abstract**

The present disclosure provides an antibody capable of suppressing proliferation of tumor cells. The antibody disclosed herein includes signal peptide of PD-1 (Programmed cell death-1) consisting of an amino acid sequence: MQIPQAPWPVVWAVLQLGWRPGW (SEQ ID NO: 1) as an antigen.

## Description

### Technical Field

The present invention relates to an antibody directed against signal peptide of PD-1 (Programmed cell death-1) and use thereof. The present invention also relates to a production method of the antibody. The present application claims priority based on Japanese Patent Application Publication No. 2021-136983 filed on August 25, 2021, the entire contents of which are incorporated herein by reference.

### Background Art

As one cancer immunotherapy, an immune checkpoint-inhibitory therapy is known. The therapy targets so-called "immune checkpoint protein" such as CTLA-4 (Cytotoxic T-lymphocyte associated antigen-4) and PD-1 known to be associated with a function of suppressing an excessive immune reaction in vivo. Such a therapy uses an inhibitor that inhibits an immune reaction suppressing mechanism in which an immune checkpoint protein is involved so as to block "mechanism for avoiding attack by immune system (also referred to as immune escape mechanism)" in which cancer cells are involved, thereby enabling attack by an immune system to cancer cells to be promoted.

It has been observed in some tumors that administration of, for example, an antibody (anti-PD-1 antibody), which is a membrane protein belonging to the immunoglobulin superfamily, against PD-1, or an antibody (anti-PD-L1 antibody) against a ligand of PD-1 called PD-L1 (programmed cell death-1 ligand-1: also referred to as B7-H1) expressed in various cells including tumor cells, as an immune checkpoint inhibitor, to patients can block the immune escape mechanism, thereby improving antitumor immune activity of immune cells (for examples, see Patent Literature 1).

### Citation List

### Patent Literature

Patent Literature 1: WO2004/004771

### Summary of Invention

### Technical Problem

As disclosed in Patent Literature 1, antibodies such as anti-PD-L1 antibody and anti-PD-L1 antibody that can be used in an immune checkpoint-inhibitory therapy are usually directed against partial protein (peptide) in the extracellular region as an antigen. This allows the antibodies to bind to PD-1 or PD-L1 and to inhibit the interaction between PD-1 and PD-L1. However, the anti-PD-L1 antibody and the anti-PD-L1 antibody may have substantially no or very low effect on improvement of the anti-tumor immune activity depending on types of tumors. Therefore, development of treatment for tumors from new approaches is being demanded.

Thus, the present invention has been made in view of the above circumstances and has one object to provide an antibody that suppresses proliferation of tumor cells. Another object is to provide a production method of the antibody.

While the conventional anti-PD-1 antibody is directed against the extracellular region of PD-1 as an antigen, the inventors of the present invention have newly produced an antibody directed against signal peptide of PD-1 as an antigen (hereinafter, also referred to as an "anti-PD-1 sp antibody"). Typically, since the signal peptide is removed before the membrane protein is transported to the cell membrane, it is assumed that PD-1 that is present in the cell membrane does not have signal peptide. Furthermore, since PD-1 is a protein that is highly expressed in immune cells (for example, T cells), it is assumed that the expression amount of PD-1 in tumor cells is relatively low. Surprisingly, however, the inventors of the present invention have studied and found that adding an anti-PD-1 sp antibody to tumor cells can suppress cell proliferation of tumor cells. This is largely different from the conventional immune checkpoint-inhibitory therapy, which aims to inhibit the interaction between PD-1 and PD-L1 (for example, binding of PD-1 and PD-L1). Therefore, the anti-PD-1 sp antibody can be expected to be used as a new approach that brings about an effect of suppressing tumor cell proliferation.

The antibody disclosed herein is an antibody directed against signal peptide of PD-1 consisting of an amino acid sequence: MQIPQAPWPVVWAVLQLGWRPGW (SEQ ID NO: 1), as an antigen. The antibody having such a structure can suppress cell proliferation of tumor cells.

The present disclosure also provides a production method of an anti-PD-1 sp antibody disclosed herein. The production method disclosed herein is a production method of an antibody directed against signal peptide of PD-1 consisting of the following amino acid sequence as an antigen:
MQIPQAPWPVVWAVLQLGWRPGW (SEQ ID NO: 1),
and the method includes preparing a conjugate as an antigen, including synthetic peptide including an entire or a part of the amino acid sequence mentioned above, a conjugate-forming polymer added to an N-terminal side or a C-terminal side of the synthetic peptide, and a carrier protein, and obtaining an antibody from a non-human mammal immunized with the antigen. Thus, an anti-PD-1 sp antibody capable of suppressing cell proliferation of tumor cells can be produced.

Furthermore, in one suitable embodiment of the production method of an anti-PD-1 sp antibody disclosed herein, at least one Cys residue is added to one end, which is not bonded to the synthetic peptide, of the conjugate-forming polymer, and the carrier protein is linked to the Cys residue via a cross-linker including a homobifunctional group or a heterobifunctional group. With such a configuration, a conjugate including a carrier protein can be easily produced.

Furthermore, in one suitable embodiment of the production method of an anti-PD-1 sp antibody disclosed herein, the conjugate-forming polymer is polyethylene glycol. Since polyethylene glycol is highly hydrophilic, water solubility of the conjugate can be improved.

### Brief Description of Drawings

FIG. 1 is a graph showing antibody titers of sera obtained from rabbits immunized with a conjugate of Example 1 as an antigen.
FIG. 2 is a graph showing antibody titers of sera obtained from rabbits immunized with a conjugate of Example 2 as an antigen.
FIG. 3 is a graph showing antibody titers of sera obtained from rabbits immunized with a conjugate of Example 3 as an antigen.

### Description of Embodiments

Hereinafter, suitable embodiments of technologies disclosed herein are described. The matters that are essential for implementation (for example, general matters associated with chemical synthesis methods of peptides, chemical synthesis methods of conjugates, and preparation of compositions) other than the matters particularly referred to in the present specification (for example, an amino acid sequence of synthetic peptide) can be understood as design matters for a person skilled in the art based on the background art in the fields of cell engineering, physiology, medical science, pharmaceutical science, organic chemistry, biochemistry, genetic engineering, protein engineering, molecular biology, genetics, and the like. The technologies disclosed herein may be implemented based on the contents disclosed herein and common technical knowledge in the art of this field. In the following description, amino acids are represented by one-letter representations in accordance with the nomenclature for amino acids indicated by the IUPAC-IUB guidelines according to circumstances.

Furthermore, the entire contents of all documents cited herein are incorporated herein by reference.

The term "synthetic peptide" as used herein refers to a peptide fragment of which a peptide chain by itself does not stably exist in nature independently and which is produced by artificial chemical synthesis or biosynthesis (namely, production based on genetic engineering) and can stably exist in predetermined system (for example, a composition including an adjuvant).

The term "peptide" as used herein refers to an amino acid polymer having a plurality of peptide bonds, typically refers to the amino acid polymer having a relatively low molecular weight such that the number of total amino acid residues is generally 100 or less (preferably 80 or less, more preferably 70 or less, and, for example, 60 or less) although not being limited by the number of amino acid residues included in the peptide chain.

The term "amino acid residue" as used herein encompasses, unless otherwise noted, an N-terminal amino acid and a C-terminal amino acid of the peptide chain.

The term "conjugate" as used herein refers to a product formed by bonding of two types or more of various compounds (for example, peptide, protein, a polymer, a cross-linker, and the like), and is encompassed by a composition.

Note here that the amino acid sequence described in this specification always includes an N-terminal side at the left side and a C-terminal side at the right side.

The term "tumor" as used herein is interpreted in a broad sense and refers to general tumors (typically, malignant tumors) including carcinoma and sarcoma or lesions in blood and hematopoietic tissues (such as leukemia and lymphoma). The term "tumor cell" has the same meaning as the term "cancer cell" and refers to a cell that forms such a tumor and typically results in abnormal proliferation (so-called malignantly transformed cells) irrelevantly to surrounding normal tissues. Therefore, unless otherwise defined, any cells classified to not normal cells but tumor cells (cancer cells) are referred to as tumor cells irrespective of the source or properties of the cells.

The term "immunized animal" as used herein refers to an animal to which an antigen is administered for the purpose of forming an antibody.

The antibody disclosed herein is an antibody (anti-PD-1 sp antibody) capable of using signal peptide of human PD-1 as an antigen. Human PD-1 is a membrane protein belonging to the immunoglobulin superfamily formed of 288 amino acid residues. PD-1 is an immune checkpoint protein expressed much in the cell membrane of immune cells such as T cells, and is bonded to a ligand (for example, PD-L1) to be involved in negative regulation of immune reactions.

The full-length sequence of the signal peptide of human PD-1 is a sequence including 23 amino acid residues in total as shown in SEQ ID NO: 1. Such an amino acid sequence is the first to 23rd amino acid sequences of the amino acid sequences constituting the human PD-1. Note here that genetic information and amino acid sequence information of PD-1 can be obtained from various data bases of public international organizations. Examples of such data bases include Universal Protein Resource (UniProt).

The anti-PD-1 sp antibody disclosed herein, when being added to tumor cells, can suppress proliferation of tumor cells. A conventional tumor cell suppression effect by an anti-PD-1 antibody is typically inhibition of the negative regulation of immune reaction by the interaction between PD-1 expressed in immune cells and ligands (for example, PD-L1) expressed in tumor cells, and the like, and promotes the suppression of proliferation of tumor cells by the activation of immune cells. However, the inventors of the present invention have found new findings that addition of the anti-PD-1 sp antibody to tumor cells suppress proliferation of tumor cells regardless of the presence of immune cells.

The production method of an anti-PD-1 sp antibody disclosed herein includes preparing a conjugate as an antigen, including c synthetic peptide including an entire or a part of the amino acid sequence set forth in SEQ ID NO: 1, a conjugate-forming polymer, and a carrier protein, and obtaining an antibody from a non-human mammal immunized with the antigen.

Synthetic peptides included in a conjugate used as an antigen typically consists of an amino acid sequence constituting the PD-1 signal peptide shown in SEQ ID NO: 1 (hereinafter, also referred to as a "PD-1 sp sequence"). The synthetic peptide may partially consist of the amino acid sequence set forth in SEQ ID NO: 1, for example, may be formed of a modified amino acid sequence modified from the PD-1 sp sequence. Examples of such modified amino acid sequence include amino acid sequences including substitution, deletion, or insertion of 1, 2 or 3 amino acid residues in the PD-1 sp sequence. Furthermore, the modified amino acid sequence can be an amino acid sequence in which 1 or 2 or more amino acid residues are added to the N-terminal side and/or the C-terminal side of the PD-1 sp sequence. Since the polyclonal antibody obtained from an immunized animal uses various parts of the synthetic peptide as an epitope, such a modified amino acid sequence can obtain an antibody using a PD-1 signal peptide as an antigen.

In order to efficiently produce an antibody using a PD-1 signal peptide as an antigen from an immunized animal, synthetic peptide preferably has high identity to the PD-1 sp sequence. For example, the identity may be 80% or more, and is more preferably 85% or more, further more preferably 90% or more, and particularly preferably 95% or more (for example, 100%).

The term "identity" as used herein has the same general interpretation in this technical field, and means the degree of sequence invariance when two or more sequences are aligned. Identity values can be calculated using, for example, various publicly available programs. Such programs include ClustalW available from the National Institute of Genetics, BLAST from the National Center for Biotechnology Information (NCBI), and the like. Although not particularly limited, when such a program is used, various conditions related to identity calculation can be used as default values.

The number of amino acid residues constituting synthetic peptide is not particularly limited, but from the viewpoint of enhancing the identity, is preferably 25 or less, and can be 24 or less. Furthermore, from the same point of view, the number of amino acid residues constituting synthetic peptide is preferably 21 or more, and more preferably 22 or more, and for example, 23.

The synthetic peptide can be easily produced according to general chemical synthesis methods. For examples, any of conventionally-known solid phase synthesis methods and liquid-phase synthesis methods may be used. The solid-phase synthesis method in which Boc (t-butyloxycarbonyl) or Fmoc (9-fluorenylmethoxycarbonyl) is used as a protective group of an amino group is suitable.

Alternatively, the synthetic peptide may be biosynthesized based on genetic engineering approaches. In other words, polynucleotide (typically DNA) of a nucleotide sequence (including the ATG start codon) encoding an amino acid sequence of desired synthetic peptide is synthesized. Then, a recombinant vector is constructed according to a host cell. The recombinant vector includes a gene construct for expression including the synthesized polynucleotide (DNA) and various regulatory elements (including a promoter, a ribosome binding site, a terminator, an enhancer, and various cis elements regulating the expression level) for expression of the amino acid sequence in the host cell.

The recombinant vector is introduced into a predetermined host cell (for example, yeast, insect cell, or plant cell) according to a general method, and the host cell or a tissue or individual including the cell is cultured under predetermined conditions. Thus, the peptide of interest can be expressed and produced in the cell. Then, the peptide is isolated from the host cell (from a culture medium when the peptide is secreted), and subjected to refolding, purification, or the like, if necessary, to obtain the peptide of interest.

For the method for constructing the recombinant vector, the methods for introducing the constructed recombinant vector into the host cell, and the like, methods that have been conventionally carried out in the art may be employed as it is. The methods per se are not characteristic of the technology disclosed herein, and therefore, detailed descriptions thereon are omitted.

Alternatively, the polypeptide of interest may be synthesized in vitro by constructing a template DNA (namely, a synthetic gene fragment including a nucleotide sequence encoding an amino acid sequence of the synthetic peptide) for a cell-free protein synthesis system, using various compounds (such as ATP, RNA polymerase, and amino acids) necessary for peptide synthesis, and employing a so-called cell-free protein synthesis system. For the cellfree protein synthesis system, for example, a research paper by Shimizu et al. (Shimizu et al., Nature Biotechnology, 19, 751-755 (2001)) and a research paper by Madin et al. (Madin et al., Proc. Natl. Acad. Sci. USA, 97(2), 559-564 (2000)) may be referred to. Based on the technologies disclosed in the research papers, many companies are already conducting contract manufacturing of peptides at the time of filing of the present application, and cellfree protein synthesis kits (for example, available from CellFree Sciences Co., Ltd., Japan) are commercially available.

A single-stranded or double-stranded polynucleotide including a nucleotide sequence encoding a synthetic peptide and/or a complementary nucleotide sequence thereto may be easily produced (synthesized) according to conventionally-known methods. In other words, by selecting codons corresponding to amino acid residues constituting a designed amino acid sequence, a nucleotide sequence corresponding to an amino acid sequence of the synthetic peptide is easily determined and provided. Once the nucleotide sequence is determined, a polynucleotide (single strand) corresponding to the desired nucleotide sequence may be easily obtained by using a DNA synthesizer or the like. Furthermore, by using the obtained single-stranded DNA as a template, a double-stranded DNA of interest may be obtained by employing any enzymatic synthesis method (typically PCR). The polynucleotide may be in the form of DNA or RNA (such as mRNA). DNA may be provided in a double-strand or single-strand form. When the DNA is provided in a single-strand form, the DNA may be a coding strand (sense strand) or a noncoding strand (antisense strand) complementary thereto.

The polynucleotide thus obtained can be used as a material for constructing a recombinant gene (expression cassette) for producing synthetic peptide in any host cell or cell-free protein synthesis system, as described above.

It is preferable in the conjugate disclosed herein that the conjugate-forming polymer is added to the N-terminal side or the C-terminal side of the synthetic peptide. As the conjugate-forming polymer, any polymers to be added to an antigen to be generally administered to immunized animals can be used without particular limitation. Examples thereof include a hydrophilic polymer, nonpeptidic linkers such as an alkyl linker and an aminohexanoyl spacer, and the like. Among them, a hydrophilic polymer is preferably used. Synthetic peptides including the entire or a part of the PD-1 sp sequence have a high proportion of hydrophobic amino acid residues and tend to aggregate in an aqueous solution. Therefore, precipitation and aggregation of the conjugate can be suppressed by adding a hydrophilic polymer to the synthetic peptide.

As the hydrophilic polymer, for example, various polyethylene glycol derivatives can be used. Among these, polyethylene glycol (PEG) can be preferably used. PEG has low antigenicity and is not easily decomposed in vivo, and thus has excellent biocompatibility. As the PEG, for example, PEG in which 2 to 24 PEG units (-CH₂-CH₂-O-) are repeated, preferably 5 to 15 PEG units are repeated, and more preferably 8 to 12 are repeated can be used. Suitable examples include PEG8 in which 8 PEG units are repeated.

The conjugate-forming polymer can be added to synthetic peptide by a conventionally known method. For example, by preparing a derivative having an amino group at one end and a carboxyl group at the other end of the conjugate-forming polymer, the conjugate-forming polymer can be treated like an amino acid residue, and therefore can be amidebonded to synthetic peptides by the above-mentioned methods (for example, the Fmoc method).

When the conjugate-forming polymer is added to the C-terminal side of the synthetic peptide, the α-amino group on the N-terminal side of the synthetic peptide is preferably acetylated. This makes it difficult for the synthetic peptide to be decomposed in vivo.

When the conjugate-forming polymer is added to the N-terminal side of the synthetic peptide, the carboxyl group on the C-terminal side of the synthetic peptide is preferably amidated. This improves water solubility.

It is preferable that the conjugate further includes a carrier protein. The carrier protein can enhance the antigenicity of the conjugate, and therefore can induce an immune response in the immunized animal and promote antibody production. The type of carrier protein is not particularly limited, but, for example, the antigenic stimulus Keyhole limpet hemocyanin (KLH) or ovalbumin (OVA) or bovine serum albumin (BSA), and the like, can be used suitably. It is preferable to use carrier proteins derived from different species from those of immunized animals.

The carrier protein can be linked to synthetic peptide or a conjugate-forming polymer via a cross-linker. As the cross-linking agent, cross-linking agents having a homobifunctional group or a heterobifunctional group and commonly used for peptide cross-linking can be preferably used. Common functional groups possessed by crosslinkers include N-hydroxysuccinimide activated esters (NHS esters), maleimides, azides and iodoacetamides. Among these, NHS esters can efficiently react with amines at neutral or higher pH to form very stable amide bonds. Furthermore, maleimide reacts selectively with SH groups, and is more excellent than amines in reactivity with SH groups in neutral conditions.

Examples of the suitable cross-linking agents having homobifunctional groups to be preferably used include N-hydroxysuccinimide (NHS), disuccinimidyl suberate (DSS), bis(sulfosuccinimidyl) suberate (BS³), dithiobis(succinic imidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), and the like. In particular, bis(sulfosuccinimidyl)suberate (BS³) can be preferably used.

Furthermore, examples of the suitable crosslinkers having heterobifunctional groups to be preferably used include N-(6-maleimidocaproyloxy)succinimide (EMCS), m-maleimidobenzoyl-N-hydroxysuccinimide ester (MBS), succinimidyl 4-[maleimide phenyl]butyrate (SMPB), succinimidyl 4-(maleimidomethyl)cyclohexane-1-carboxylate (SMCC), N-(y-maleimidobutyroxy)succinimide ester (GMBS), m-maleimidopropionic acid-N-hydroxy Succinimide ester (MPS), N-succinimidyl (4-iodoacetyl) aminobenzoate (SIAB), and the like. In particular, EMCS and MBS having NHS ester and maleimide as reactive functional groups can be preferably used.

In one suitable example of a conjugate including a carrier protein, a conjugate-forming polymer is added to the N-terminal side or the C-terminal side of the synthetic peptide, and a Cys residue is added to one end that is not bonded to the synthetic peptide in the ends of the conjugate-forming polymer, and a carrier protein is bonded to the Cys residue via a cross-linking agent. Since the PD-1 sp sequence does not include a Cys residue, according to such a configuration, the carrier protein and the added Cys residue can be easily combined with each other by a cross-linking agent having an SH group-selective functional group (for example, maleimide).

Next, a non-human mammal is immunized with the conjugate prepared above as an antigen to obtain an anti-PD-1 sp antibody from the immunized non-human mammal.

Examples of the mammals to be immunized include experimental animals such as guinea pigs, rats, mice, rabbits, and sheep. For obtaining monoclonal or polyclonal antibodies, rats, mice, and rabbits are suitable. For immunization methods, any administration route such as subcutaneous, intradermal, intraperitoneal, intravenous, intramuscular, and the like, may be used, but, mainly, subcutaneous, intradermal, intraperitoneal, and intravenous injections are preferable. In addition, various methods can be used without any particular restrictions on the immunization interval, immunization dose, and the like, but, for example, a method of performing immunization about 2 to 10 times at intervals of 1 to 2 weeks, and about 2 to 7 days after the final immunization, and collecting samples from living bodies is often used. Furthermore, although the amount of antigen to be administered at one time is not limited, but, for example, it is preferable to use about 25 to 300 µg per rabbit. Also, although not particularly limited, it is preferable that in the first immunization, the conjugate mixed well with an adjuvant (for example, FCA (Freund's complete adjuvant)) is administered to immunized animals to proliferate immune cells, and the conjugate well mixed with an adjuvant (for example, FCA or FIA (Freund's incomplete adjuvant)) is administered again at interval of 1 to 2 weeks. Thus, antiserum including monoclonal antibodies or polyclonal antibodies can be suitably obtained. Purification of the desired monoclonal antibody or polyclonal antibody of interest can be performed by well-known methods such as affinity chromatography, ion exchange chromatography, gel filtration, and salting out with ammonium sulfate.

The antibody thus obtained is an anti-PD-1 sp antibody that can use the PD-1 signal peptide including the amino acid set forth in SEQ ID NO: 1 as an antigen. The antibody titer of the obtained antibody directed against the PD-1 signal peptide can be evaluated by, for example, an ELISA (Enzyme-Linked ImmunoSorbent Assay) method using a microplate on which the PD-1 signal peptide is immobilized.

Furthermore, a monoclonal antibody directed against the PD-1 signal peptide as an antigen can be obtained by a well-known method. For example, B-cells are harvested from the spleen of a mammal immunized with the conjugate and fused with myeloma cells to produce hybridoma. Then, by screening the prepared hybridomas and selecting hybridomas that produce anti-PD-1 sp antibody, it is possible to obtain monoclonal antibodies directed against the PD-1 signal peptide as an antigen.

The anti-PD-1 sp antibody can be suitably used as an active component of a composition (that is, a pharmaceutical antitumor composition such as an antitumor agent) for suppressing (or inhibiting) the proliferation of tumor cells. The anti-PD-1 sp antibody may include a variety of pharmacologically (pharmaceutically) acceptable carriers depending on the form of use as long as the carriers do not lose the anti-tumor activity. For example, carriers commonly used as diluents, excipients, and the like, in peptide medicines can be applied.

Various organic solvents such as water and physiological buffers may be typically used although they may vary depending on the applications and forms of antitumor compositions. An aqueous solution of alcohol (ethanol and the like) with appropriate concentration, glycerol, non-drying oil such as olive oil may be used. Alternatively, liposome may be used. Furthermore, examples of the secondary components that can be included in the anti-tumor composition include various fillers, extenders, binders, moisturizers, surfactants, dyes, perfumes, and the like.

Typical forms of antitumor compositions (antitumor agents) include liquids, suspensions, emulsions, aerosols, foams, granules, powders, tablets, capsules, ointments, aqueous gels, and the like. Furthermore, for use in injection, and the like, the form may be a lyophilized product or granulated product for preparing a drug solution by being dissolved in physiological saline, an appropriate buffer solution (for example, PBS), or the like, immediately before use.

The process itself for preparing various forms of compositions (drugs) using the anti-PD-1 sp antibody (main component) and various carriers (subcomponents) as materials may follow conventionally known methods, and the methods per se do not characterize the technology disclosed herein, and therefore, the detailed description thereof will be omitted. Sources of detailed information on prescription include, for example, Comprehensive Medicinal Chemistry, edited by Corwin Hansch, published by Pergamon Press (1990). The entire contents of this book are incorporated herein by reference.

The cells to which the antitumor composition (antitumor peptide) disclosed herein is applied are not particularly limited as long as the cells are tumor cells (cancer cells), but can be applied to various types of tumor cells generated in humans. Examples of the cells include various types of squamous cell carcinoma and adenocarcinoma. Examples of the cells include cancer cells of breast cancer, pancreatic cancer, prostate cancer, lung cancer (non small cell lung cancer and small cell lung cancer), and the like, or cells constituting skin cancer such as melanoma and basal cell cancer, neuroblastic tumor (neuroblastoma), retinoblastoma, pheochromocytoma, and other types of cell tumors.

The antitumor composition can be used by a method and at a dose according to the form and purpose thereof as in conventional peptide formulations. For example, the antitumor composition may be administered as a solution at a desired dose to a diseased site (typically malignant tumor tissue) of a patient (namely, a living body) by an intravenous, intramuscular, subcutaneous, intradermal, or intraperitoneal injection. Alternatively, the antitumor composition in the form of solid such as tablets or gel or aqueous jelly such as ointment may be administered directly to a certain tissue (namely, a diseased site of tissue or organ including tumor cells). Alternatively, the antitumor composition in the form of solid such as tablets can be orally administered. In case of oral administration, it is preferable to encapsulate or use a protective (coating) material in order to suppress decomposition by digestive enzymes in the digestive tracts.

Alternatively, to tumor cells (including cell mass or tissue or organ excised from a living body) cultured in vitro, an appropriate amount of the antitumor composition disclosed herein (in other words, an appropriate amount of the anti-PD-1 sp antibody) may be provided to a medium of cultured cells (such as tissue) to be treated at least once. The amount per dose and frequency of provision are not particularly limited and may vary according to the conditions such as the type of the cultured tumor cells, cell density (cell density at the beginning of culture), the number of subcultures, culture conditions, and the type of media.

Hereinafter, some specific examples relating to technologies of the present invention are described. However, it is not intended to limit the present invention to the specific examples.

### <Preparation of conjugate>

The conjugates of Examples 1 to 3 were synthesized as antigens for immunizing rabbits. The structures of the conjugates of Examples 1 to 3 are shown in Table 1. Firstly, for production of the conjugate of Example 1, synthetic peptide consisting of the amino acid sequence set forth in SEQ ID NO: 1, PEG8 added to the C-terminal side of the synthetic peptide, and a Cys residue added to the end of the PEG8 were synthesized. Such synthesis was carried out according to the manual of the peptide synthesizer. By using a compound having a carboxyl group at one end and an amino group at the other end, PEG8 was bonded to an adjacent amino acid via an amide bond. Next, by the maleimide method, keyhole limpet hemocyanin (KLH) as the carrier protein, and the Cys residue synthesized above are bonded to each other via a cross-linking agent, N-(6-maleimidocaproyloxy)succinimide (EMCS). Thus, the conjugate of Example 1 was obtained. The conjugate of Example 2 was produced in the same manner as the method of producing the conjugate of Example 1 except that the amino acid sequence constituting the synthetic peptide was changed to the amino acid sequence set forth in SEQ ID NO: 2. The conjugate of Example 3 was produced in the same manner as the method of producing the conjugate of Example except that the amino acid sequence constituting the synthetic peptide was changed to the amino acid sequence set forth in SEQ ID NO: 3 and the amino group at the N-terminal side of the synthetic peptide was acetylated. Note here that since the mode of use of the peptide synthesizer itself does not characterize the present disclosure, detailed description thereof is omitted.

The amino acid sequence set forth in SEQ ID NO: 2 is the full-length sequence of the full-length signal peptide of human PD-L1. Furthermore, the amino acid sequence set forth in SEQ ID NO: 3 is a partial sequence of the signal peptide of human CTLA-4, and corresponds to the amino acid sequence from 4th to 18th of the full-length signal peptide of human CTLA-4 shown in SEQ ID NO: 4.

### [Table 1]

**Table 1**

| | Structure of conjugate |
|---|---|
| Example 1 | NH₂₋MQIPQAPWPVVWAVLQLGWRPGW-(PEG8)-C-KLH |
| Example 2 | NH₂₋MRIFAVFIFMTYWHLLNA-(PEG8)-C-KLH |
| Example 3 | Acetyl-LGFQRHKAQLNLATR-(PEG8)-C-KLH |

### <Production of antibody directed against conjugate as antigen>

Antibodies were prepared by administering (immunizing) the obtained conjugate as an antigen to Japanese white rabbits. Furthermore, rabbit blood was collected before and after immunization for evaluation of antibody titer. Specifically, on day 1 (hereinafter, the number of days based on this day will be described), 5 ml of test blood collection was carried out before administration of the conjugate, and preimmune serum were prepared. On the same day, a composition obtained by mixing 0.2 mg of the conjugate and an equal volume of FCA was intradermally administered to the above rabbits. Then, on day 8 and day 15, a composition obtained by mixing 0.2 mg of the above conjugate and an equal volume of FCA were intradermally administered to the rabbits. On day 22, 0.025 mg of conjugate was administered intravenously to the rabbits. On day 29, 5 ml of blood was collected from the rabbits to prepare postimmune serum.

### <Evaluation of antibody titer of serum>

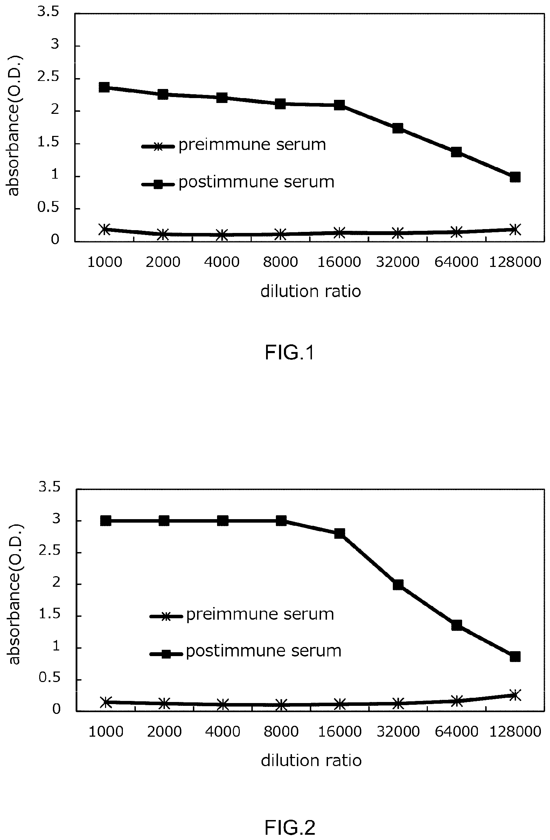

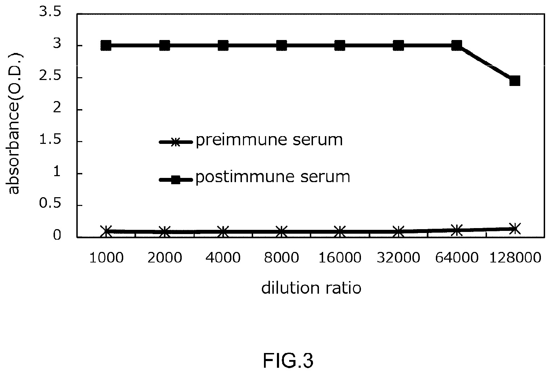

Antibody titers of preimmune serum and postimmune serum were evaluated by the ELISA method. Firstly, synthetic peptide portion included in the conjugate is dissolved in PBS (Phosphate-Buffered Saline) of pH7.2 so as to be 5 µg/ml, 100 µl each of which was added to each well of the microplate (manufactured by NalgeNunc, cat#442404, a flat-bottom 96-well), followed by incubation for 2 hours at room temperature. Next, after the liquid in each well was removed, washing was carried out three times with PBS (washing liquid) including 0.2% Tween-20 (Polyoxyethylene(20) Sorbitan Monolaurate manufactured by Fujifilm Wako Pure Chemical Industries, Ltd.). Then, the washing solution was added to each well, and incubation was carried out overnight at 4°C. After incubation, preimmune serum and postimmune serum were diluted 1000-fold, 2000-fold, 4000-fold, 8000-fold, 16000-fold, 32000-fold, 64000-fold, and 128000-fold, respectively, with PBS (diluent) including 0.05% Tween-20. After the washing solution in each well was removed, 100 µl of each diluent was added to each of the other wells, incubation was carried out at 37°C for 30 minutes, and then incubation was carried out at room temperature for 15 minutes. After the liquid in each well was removed, washing was carried out three times with the washing solution, and goat f(ab)'2 anti-rabbit IgG's HRP conjugate (manufactured by MP Biomedicals, LLC-Cappel Products) that had been diluted 5000-fold with the diluent was added to each well in an amount of 100 µl each, followed by incubation at 37°C for 30 minutes, and further incubation was carried out at room temperature for 15 minutes. After removing the liquid of each well, washing was carried out three times with the washing solution, and 100 µl each of substrate solution obtained by dissolving 10 mg of OPD (O-phenylene diamine, manufactured by SIGMA) in 25 ml of citrate-phosphate buffer, and adding 5 µl of hydrogen peroxide to each well. Thereafter, color development was performed at room temperature for 20 minutes, and 100 µl each of 1 M sulfuric acid was added to each well to stop color development. Then, the absorbance of each well at 490 nm was measured using an Immno Reader. The results are shown in FIGs. 1 to 3. FIG. 1 is a graph showing antibody titers of sera obtained from rabbits with the conjugate of Example 1 as an antigen. FIG. 2 is a graph showing antibody titers of sera obtained from rabbits with the conjugate of Example 2 as an antigen. FIG. 3 is a graph showing antibody titers of sera obtained from rabbits immunized with the conjugate of Example 3 as an antigen. Note here that when the absorbance exceeds the measurement upper limit of the Immno Reader (when the absorbance exceeds 3.0), the absorbance is indicated as 3.0.

As shown in FIGs. 1 to 3, when immunization with any one of the conjugates of Examples 1 to 3 was carried out, postimmune sera showed remarkably higher absorbance than preimmune sera. This shows that an antiserum including an antibody (anti-PD-1 sp antibody) against the full-length signal peptide sequence of PD-1 consisting of the amino acid sequence set forth in SEQ ID NO: 1, an antiserum including an antibody against the full-length signal peptide sequence of PD-L1 consisting of the amino acid sequence set forth in SEQ ID NO: 2, and an antiserum including an antibody against a part of the signal peptide sequence of CTLA-4 consisting of the amino acid sequence set forth in SEQ ID NO: 3 are obtained.

### <Evaluation of suppression of cell proliferation of antiserum>

A proliferation suppression effect of antiserum obtained above against tumor cells was evaluated. In this test, HeLa cells (an established cell line derived from human cervical cancer cells) were used as tumor cells. As a normal culture medium for HeLa cells, a DMEM (Dulbecco's modified Eagle's medium (manufactured by Fujifilm Wako Pure Chemical Industries, Ltd., Cat No. 043-30085)) containing 10% FBS (fetal bovine serum (manufactured by Hyclone)), 2 mM L-glutamine, and antibiotics (50 units/mL penicillin G, and 50 µg/mL streptomycin) was used.

Furthermore, the antiserum was diluted with an antibiotic-free culture medium to be 20 vol%, and 20% antiserum-containing media of Examples 1 to 3 were prepared, respectively.

Firstly, HeLa cells were suspended in a culture medium so as to be 1 × 10⁴ cells/100 µL, and 100 µL each of such a suspension was added to each well of a 96-well plate (i.e., 1 × 10⁴ cells/well). After that, incubation was carried out under 5% CO₂ conditions at 37°C for about 24 hours. Then, after removing the medium in each well, 90 µL of 20% antiserum-containing medium or 90 µL of normal culture medium was added instead, and incubation was further carried out under 5% CO₂ conditions at 37°C for about 24 hours. At this time, in each Example, the number of wells to which 20% antiserum-containing medium was three, and the number of wells to which the normal culture medium was added was three.

After about 24 hours of incubation, the medium in each well was removed and 100 µL of culture medium was added. Furthermore, 10 µL each of a cell proliferation measuring reagent "Cell Counting Kit-8" (manufactured by Dojindo Laboratories) containing a "watersoluble tetrazolium salt (WST-8)" as a color development reagent was added to each well. At this time, a blank well was set by adding 100 µL of culture medium and 10 µL of the color development reagent to a well in which no cells were seeded. After that, incubation was carried out under 5% CO₂ conditions at 37°C for 1.5 hours. Then, the absorbance of each well at 450 nm was measured using a microplate reader. The system incubated with 20% antiserum-containing medium is referred to as "antiserum (+)", and the system incubated with normal culture medium instead of 20% antiserum-containing medium is referred to as "antiserum (-)". In each Example, the average value of absorbance at 450 nm in each system was determined. At this time, the average value was calculated by subtracting the absorbance value at 450 nm in the blank. In each Example, the cell survival rate (%) was obtained by calculating "100 x (average value of absorbance at 450 nm for antiserum (+)) / (average value of absorbance at 450 nm for antiserum (-))". The results are shown in Table 2.

### [Table 2]

**Table 2**

| | Antiserum (-) | Antiserum (+) | Survival rate |
|---|---|---|---|
| Example 1 | 1.984 | 1.467 | 74% |
| Example 2 | 1.996 | 2.014 | 101% |
| Example 3 | 2.043 | 2.003 | 98% |

As shown in Table 2, it is shown that when the antiserum of Example 1 was added, the survival rate is much lower than in Examples 2 and 3. That is, it is shown that addition of the anti-PD-1 sp antibody to tumor cells (HeLa cells) suppresses proliferation of tumor cells. In other words, it is said that the anti-PD-L1 antibody has an antitumor activity (tumor cell proliferation suppression activity).

This can be said to be a surprising result that antitumor activity can be exhibited when the anti-PD-1 sp antibody disclosed herein is added to tumor cells even in the absence of immune cells, while conventional anti-PD-1 antibodies inhibit the interaction between PD-1 expressed in immune cells (for example, T cells) and PD-L1 expressed in tumor cells (for example, binding inhibition of PD-1 and PD-L1) to exhibit the antitumor activity.

In the above, specific examples of the technology disclosed herein have been described in detail, but these are merely examples and do not limit the scope of the claims. The technology described in the claims includes various modifications and changes of the specific examples illustrated above.

As described above, the anti-PD-1 sp antibody disclosed herein can suppress (or inhibit) proliferation of tumor cells. Therefore, an antitumor composition (antitumor agent) that suppresses proliferation of at least one type of tumor cells by using the anti-PD-1 sp antibody can be provided.

[Sequence Listing]

## Claims

1. An antibody directed against signal peptide of PD-1 (Programmed cell death-1) consisting of the following amino acid sequence as an antigen:
MQIPQAPWPVVWAVLQLGWRPGW (SEQ ID NO: 1).

2. A production method of an antibody directed against signal peptide of PD-1 (Programmed cell death-1) consisting of the following amino acid sequence as an antigen:
MQIPQAPWPVVWAVLQLGWRPGW (SEQ ID NO: 1),
the method comprising:
preparing a conjugate as an antigen, including synthetic peptide including an entire or a part of the amino acid sequence, a conjugate-forming polymer added to an N-terminal side or a C-terminal side of the synthetic peptide, and a carrier protein, and
obtaining an antibody from a non-human mammal immunized with the antigen.

3. The production method according to claim 2, wherein at least one Cys residue is added to one end, which is not bonded to the synthetic peptide, of the conjugate-forming polymer, and the carrier protein is linked to the Cys residue via a cross-linker having a homobifunctional group or a heterobifunctional group.

4. The production method according to claim 2 or 3, wherein the conjugate-forming polymer is polyethylene glycol.
